# EUROPEAN PATENT SPECIFICATION

(11) **EP 4 298 932 B1**
(45) Date of publication and mention of the grant of the patent: **04.09.2024**
(21) Application number: 22826786.0
(22) Date of filing: 13.05.2022
(51) Int. Cl.: A24F 40/46, A24F 40/44

(54) **HEAT GENERATING ELEMENT, ATOMIZING ASSEMBLY, AND ELECTRONIC ATOMIZING DEVICE**
HEIZKÖRPER, ZERSTÄUBUNGSANORDNUNG UND ELEKTRONISCHE ZERSTÄUBUNGSVORRICHTUNG
ÉLÉMENT DE GÉNÉRATION DE CHALEUR, ENSEMBLE D'ATOMISATION ET DISPOSITIF D'ATOMISATION ÉLECTRONIQUE

(43) Date of publication of application: 03.01.2024
(73) Proprietor: Shenzhen Smoore Technology Limited, Shenzhen Guangdong 518102 (CN)
(72) Inventor: LV, Ming, Shenzhen, Guangdong 518102 (CN); WANG, Wei, Shenzhen, Guangdong 518102 (CN); ZHU, Mingda, Shenzhen, Guangdong 518102 (CN); HUANG, Rongji, Shenzhen, Guangdong 518102 (CN); FENG, Jianming, Shenzhen, Guangdong 518102 (CN)
(74) Representative: Herrero & Asociados, S.L.
(86) International application number: PCT/CN2022/092859
(87) International publication number: WO 2023/216263

(56) References cited:
- WO-A1-2017/214879
- WO-A1-2019/035056
- WO-A1-2021/056195
- WO-A1-2021/120802
- WO-A1-2021/217292
- WO-A1-2021/244543
- WO-A2-2014/037794
- CN-A- 109 414 078
- CN-A- 113 615 887
- CN-A- 113 768 192
- CN-A- 113 768 192
- CN-A- 113 826 962
- CN-A- 113 951 572
- CN-A- 114 287 676
- CN-A- 114 451 586
- CN-U- 216 453 382
- US-A1- 2022 117 305

## Description

### TECHNICAL FIELD

The present disclosure relates to the field of atomization technologies, and in particular, to a heating body, an atomization assembly, and an electronic atomization device.

### BACKGROUND

A typical electronic atomization device includes a heating body, a battery, and a control circuit, etc. The heating body is a core component of the electronic atomization device, and both an atomizing effect of the electronic and using experience provided by the atomization device are depended on characteristics of the heating body.

An existing heating body has a risk of being corroded in a strong corrosive aerosol-generating substance and thereby has a shorter service life. Different approaches to address this risk are described in WO 2012/120802A, WO 2019/035056, WO 2014/037794 A or WO2012/056195 A.

### SUMMARY

The present disclosure provides a heating body, an atomization assembly, and an electronic atomization device, to resolve the technical problem that the service life of a heating body is short in the related art.

To solve the above technical problem, a first technical solution provided in the present disclosure is to provide a heating body, applied to an electronic atomization device and configured to atomize an aerosol-generating substance, wherein the heating body includes a liquid-guiding substrate; a heating material layer, arranged on a first surface of the liquid-guiding substrate, wherein the heating material layer is a resistance heating material, and includes a heating portion wherein a portion of the liquid-guiding substrate (111), where the heating portion (1121) is arranged, is defined as a heating region and a connection portion wherein another portion of the liquid-guiding substrate (111), where the connection portion (1122) is arranged, is defined as an electrode region; a first protecting film, at least partially arranged on the surface of the heating portion away from the liquid-guiding substrate, wherein the material of the first protecting film is non-conductive and resistant to corrosion of the aerosol-generating substance; and a second protecting film, at least partially arranged on the surface of the connection portion away from the liquid-guiding substrate, wherein the material of the second protecting film is conductive and resistant to the corrosion of the aerosol-generating substance.

To solve the above technical problem, a second technical solution provided in the present disclosure is to provide an atomization assembly, including a liquid storage cavity and a heating body, configured to store a liquid aerosol-generating substance; the above heating body; and the heating body is in fluid communication with the liquid storage cavity.

To solve the above technical problem, a third technical solution provided in the present disclosure is to provide an electronic atomization device, including the above atomization assembly and a power supply assembly electrically connected to the heating body.

The present disclosure may have the following beneficial effects. Different from the related art, the present disclosure discloses a heating body, an atomization assembly, and an electronic atomization device. The heating body includes a liquid-guiding substrate, a heating material layer, a first protecting film, and a second protecting film. The liquid-guiding substrate includes a heating region and an electrode region. The heating material layer is arranged on a first surface of the liquid-guiding substrate and includes a heating portion arranged in the heating region and a connection portion arranged in the electrode region. The first protecting film is arranged on the surface of the heating portion away from the liquid-guiding substrate, and the material of the first protecting film is non-conductive and resistant to corrosion of an aerosol-generating substance. The second protecting film is arranged on the surface of the connection portion away from the liquid-guiding substrate, and the material of the second protecting film is a conductive and resistant to the corrosion of the aerosol-generating substance. The heating region and the electrode region of the heating material layer are protected with different protecting films, such that the heating material layer is prevented from being corroded by the aerosol-generating substance. In this way, a service life of the heating material layer is improved.

### BRIEF DESCRIPTION OF THE DRAWINGS

To describe the technical solutions in the embodiments of the present disclosure more clearly, the accompanying drawings required for describing the embodiments will be briefly illustrated. The accompanying drawings in the following description show merely some embodiments of the present disclosure, and a person of ordinary skill in the art may still derive other accompanying drawings from these accompanying drawings without creative efforts.
FIG. 1 is a structural schematic view of an electronic atomization device according to some embodiments of the present disclosure.
FIG. 2 is a structural schematic view of an atomization assembly of the electronic atomization device according to some embodiments of the present disclosure.
FIG. 3a is a structural schematic view of a heating body according to a first embodiment of the present disclosure.
FIG. 3b is a structural schematic top view of the heating body in FIG. 3a.
FIG. 4 is a structural schematic view of a liquid-guiding substrate of the heating body in FIG. 3a.
FIG. 5 is a structural schematic view of the heating body according to a second embodiment of the present disclosure.
FIG. 6 is a structural schematic view of the heating body according to a third embodiment of the present disclosure.
FIG. 7 is a structural schematic view of the heating body according to a fourth embodiment of the present disclosure.
FIG. 8 is a structural schematic view of the heating body according to a fifth embodiment of the present disclosure.
FIG. 9 is a structural schematic view of the heating body according to a sixth embodiment of the present disclosure.
FIG. 10 is a schematic view of a process of a wet burning experiment on the heating body according to the present disclosure.

### DETAILED DESCRIPTION

The technical solutions in the embodiments of the present disclosure are clearly and completely described below with reference to the accompanying drawings in the embodiments of the present disclosure. The described embodiments are merely some rather than all of the embodiments of the present disclosure. All other embodiments obtained by a person of ordinary skill in the art based on the embodiments of the present disclosure without creative efforts shall fall within the protection scope of the present disclosure.

In the following description, for the purpose of illustration rather than limitation, a specific detail such as a specific system structure, an interface, technology, or the like, are proposed to thoroughly understand the present disclosure.

The terms "first", "second", and "third" in the present disclosure are merely intended for a purpose of description, and shall not be understood to indicate or imply relative significance or implicitly indicate the number of indicated technical features. Therefore, features defining with the terms "first", "second", and "third" can explicitly or implicitly include at least one of the features. In the description of the present disclosure, "a plurality of" means at least two, such as two, and three, etc., unless it is specifically defined otherwise. All directional indications (for example, upper, lower, left, right, front, and rear) in the embodiments of the present disclosure are only used for explaining relative position relationships, movement situations, or the like between various components under a specific posture (as shown in the accompanying drawings). When the specific posture changes, the directional indications change accordingly. In the embodiments of the present disclosure, the terms "include", "have", and any variant thereof are intended to cover a non-exclusive inclusion. For example, a process, a method, a system, a product, or a device including a series of steps or units is not limited to the listed steps or units, but alternatively includes a step or a unit which is not listed, or further alternatively includes another step or component intrinsic to the process, the method, the product, or the device.

The present disclosure is described in detail below with reference to the accompanying drawings and the embodiments.

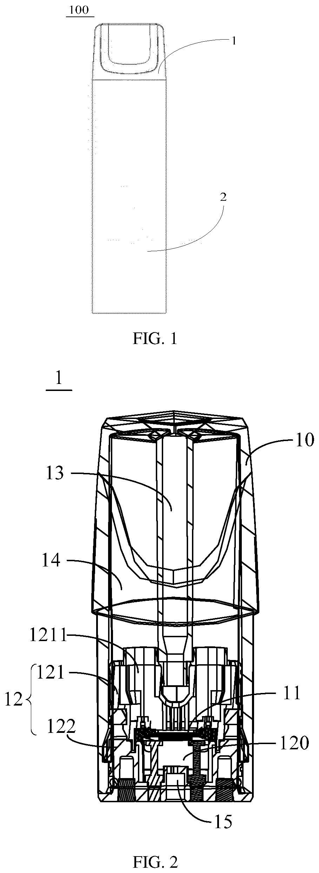

As shown in FIG. 1, FIG. 1 is a structural schematic view of an electronic atomization device according to some embodiments of the present disclosure.

In this embodiment, an electronic atomization device 100 is provided. The electronic atomization device 100 may be configured to atomize an aerosol-generating substance. The electronic atomization device 100 includes an atomization assembly 1 and a power supply assembly 2 electrically connected to the atomization assembly 1.

The atomization assembly 1 is configured to store an aerosol-generating substance and atomize the aerosol-generating substance to form aerosol for a user to inhale. The atomization assembly 1 may be applied to different fields such as medical care, cosmetology, and recreation inhalation. In a specific embodiment, the atomization assembly 1 may be applied to an electronic aerosol atomization device to atomize the aerosol-generating substance and generate the aerosol for an inhaler to inhale, and all the following embodiments are described with taking the recreation inhalation as an example.

For a specific structure and functions of the atomization assembly 1, reference may be made to the specific structure and the functions of the atomization assembly 1 described in the following embodiments, same or similar technical effects may be implemented, and details are not repeated herein.

The power supply assembly 2 includes a battery (not shown in the drawings) and a controller (not shown in the drawings). The battery is configured to supply electric energy for operation of the atomization assembly 1, such that the atomization assembly 1 atomizes the aerosol-generating substance to form the aerosol. The controller is configured to control the operation of the atomization assembly 1. The power supply assembly 2 further includes a component such as a battery holder, an airflow sensor, etc.

The atomization assembly 1 and the power supply assembly 2 may be integrally arranged or may be detachably connected to each other, which may be designed according to a specific requirement.

The power of the electronic atomization device generally does not exceed 10 W, and ranges from 6 W to 8.5 W. The voltage of the battery adopted by the electronic atomization device ranges from 2.5 V to 4.4 V. For a closed electronic atomization device (an electronic atomization device without a requirement for the user to autonomously inject an aerosol-generating substance), the voltage of a battery of the closed electronic atomization device ranges from 3 V to 4.4 V. The electronic atomization device of the present disclosure is not limited to these parameters.

As shown in FIG. 2, FIG. 2 is a structural schematic view of an atomization assembly of the electronic atomization device according to some embodiments of the present disclosure.

The atomization assembly 1 includes a housing 10, a heating body 11, and an atomization base 12. The atomization base 12 includes a mounting cavity (not marked in the drawings), and the heating body 11 is arranged in the mounting cavity. The heating body 11 is arranged in the housing 10 together with the atomization base 12. The housing 10 is provided with a vapor outlet channel 13. A liquid storage cavity 14 is defined by an inner surface of the housing 10, an outer surface of the vapor outlet channel 13, and the top surface of the atomization base 12 corporately. The liquid storage cavity 14 is configured to store a liquid aerosol-generating substance. The heating body 11 is electrically connected to the power supply assembly 2, so as to atomize the aerosol-generating substance to generate the aerosol.

The atomization base 12 includes an upper base 121 and a lower base 122, and the mounting cavity is defined by the upper base 121 and the lower base 122 corporately. An atomization cavity 120 is defined by an atomization surface of the heating body 11 and the cavity walls of the mounting cavity corporately. A liquid supplying channel 1211 is provided on the upper base 121, and the liquid supplying channel 1211 is in fluid communication with the mounting cavity. The aerosol-generating substance in the liquid storage cavity 14 flows into the heating body 11 through the liquid supplying channel 1211, i.e., the heating body 11 is in fluid communication with the liquid storage cavity 14. An air inlet channel 15 is provided on the lower base 122. The external air enters the atomization cavity 120 through the air inlet channel 15, and carries the aerosol atomized by the heating body 11 to flow to the vapor outlet channel 13. The user inhales the aerosol through the end opening of the vapor outlet channel 13.

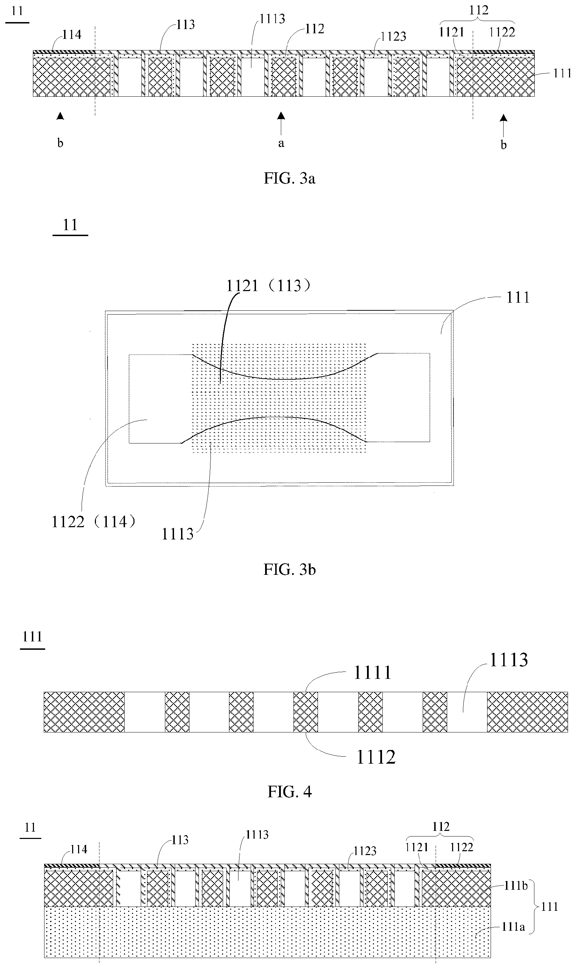

As shown in FIG. 3a to FIG. 4, FIG. 3a is a structural schematic view of a heating body according to a first embodiment of the present disclosure, FIG. 3b is a structural schematic top view of the heating body in FIG. 3a, and FIG. 4 is a structural schematic view of a liquid-guiding substrate of the heating body in FIG. 3a.

The heating body 11 includes a liquid-guiding substrate 111, a heating material layer 112, a first protecting film 113, and a second protecting film 114. The liquid-guiding substrate 111 has a structure supporting function. The heating material layer 112 is a resistance heating material. The liquid-guiding substrate 111 includes a first surface 1111 and a second surface 1112 arranged opposite to the first surface 1111. The first surface 1111 of the liquid-guiding substrate 111 includes a heating region a and an electrode region b. The heating material layer 112 is arranged on the first surface 1111 of the liquid-guiding substrate 111, and includes a heating portion 1121 arranged in the heating region a and a connection portion 1122 arranged in the electrode region b. The connection portion 1122 serves as an electrode, and is configured to be electrically connected to the power supply assembly 2. The first protecting film 113 is at least partially arranged on the surface of the heating portion 1121 away from the liquid-guiding substrate 111, and the material of the first protecting film 113 is a non-conductive and resistant to corrosion of the aerosol-generating substance. The second protecting film 114 is at least partially arranged on the surface of the connection portion 1122 away from the liquid-guiding substrate 111, and the material of the second protecting film 114 is a conductive and resistant to the corrosion of the aerosol-generating substance.

Based on the foregoing arrangement, different regions of the heating material layer 112 are protected by different protecting films, respectively, so that the corrosion of the aerosol-generating substance to the heating portion 1121 and the connection portion 1122 is effectively prevented, and accordingly a service life of the heating material layer 112 is improved.

In an embodiment, the first protecting film 113 covers the entire heating portion 1121, to prevent the corrosion of the aerosol-generating substance to the entire heating portion 1121, so that the entire heating portion 1121 is protected, and thereby the service life of the heating body 11 is improved.

In an embodiment, the second protecting film 114 covers the entire connection portion 1122, to prevent the corrosion of the aerosol-generating substance to the entire connection portion 1122, so that the entire connection portion 1122 is protected, and thereby the service life of the heating body 11 is improved.

In an embodiment, an opening (not shown in the drawings) is provided on the second protecting film 114 to expose a part of the connection portion 1122. The exposed connection portion 1122 is configured to be in contact with a conducting member (not marked in the drawings). Based on the arrangement, the contact resistance between the conducting member and the connection portion 1122 is reduced. It may be understood that, the connection portion 1122 is electrically connected to the power supply assembly 2 through the conducting member (not marked in the drawings). The conducting member may be an ejector pin or a spring pin.

In an embodiment, the material of the first protecting film 113 is ceramic or glass. Since the material of the heating material layer 112 is metal, the thermal expansion coefficient of the ceramic or the glass matches the thermal expansion coefficient of the metal heating material layer 112, and the adhesion force of the ceramic or the glass matches the adhesion force of the metal heating material layer 112. Therefore, the ceramic or the glass is configured as the first protecting film 113, and the first protecting film 113 may be difficult to fall off the heating portion 1121, so that the heating portion can be well protected.

When the material of the first protecting film 113 is the ceramic, the material of the ceramic may be one or more of aluminum nitride, silicon nitride, aluminum oxide, silicon oxide, silicon carbide, or zirconium oxide, which is selected as required. As shown in Table 1 and Table 2, compared with a case where a stainless steel resistant to the corrosion of the aerosol-generating substance is served as a protecting film to protect the heating portion 1121, the ceramic is served as the first protecting film 113 to protect the heating portion 1121, and the first protecting film 113 has higher heat conduction performance and a smaller contacting angle to the aerosol-generating substance. The higher heat conduction performance of the first protecting film 113 may conduct the heat generated by the heating portion 1121 to the aerosol-generating substance more efficiently, which improves the atomization efficiency of the heating portion 1121. The smaller contact angle of the first protecting film 113 brings stronger wettability of the aerosol-generating substance on the surface of the first protecting film and accordingly a higher transmission efficiency of the aerosol-generating substance, which further improves the atomization efficiency of the heating portion 1121. Experiments was performed for the heating body 11 with the first protecting film 113 (the first protecting film 113 adopts the aluminum nitride) as a protecting film of the heating portion 1121 and a heating body in the related art (which adopts the stainless steel as the protecting film). The experiment conditions are as follows: the constant power of 6.5 W, and 3s of inhalation and then stopped for 27s. The atomization amount of the heating body 11 provided in the present disclosure is 7.2 mg/puff, and the atomization amount of the heating body in the related art is 6.2 mg/puff, which proves that the atomization amount may be apparently improved by adopting the ceramic as the first protecting film 113. The thermal conductivities of some materials are shown in Table 1; and contacting angles of these materials are shown in Table 2.

**Table 1: Thermal conductivities of materials**

| Material | Protecting film | | | | |
|---|---|---|---|---|---|
| | Stainless steel | Aluminum nitride | Silicon nitride | Aluminum oxide | Silicon carbide |
| Thermal conductivity (W/m·K) | 15 | 180 | 27 | 45 | 200 |

**Table 2: Contact angles of materials**

| Material | Stainless steel | Aluminum nitride | Silicon nitride | Aluminum oxide | Silicon carbide |
|---|---|---|---|---|---|
| Contact angle | 45° | 20° | 20° | 23° | 22° |

In an embodiment, the thickness of the first protecting film 113 ranges from 10 nm to 1000 nm. When the thickness is less than 10 nm, the first protecting film 113 may be difficult to achieve a protecting effect, and the aerosol-generating substance may penetrate the first protecting film 113 and corrode the heating portion 1121 due to a poor density of the thin first protecting film. When the thickness of the first protecting film 113 is greater than 1000 nm, the first protecting film 113 is easily cracked due to a thermal shock and loses the protecting effect due to excessively great stress.

In an embodiment, the thickness of the second protecting film 114 ranges from 10 nm to 2000 nm. When the thickness is less than 10 nm, the second protecting film 114 may be difficult to achieve a protecting effect, since the aerosol-generating substance may penetrate the second protecting film 114 and corrode the connection portion 1122 due to a poor density of the thin second protecting film. When the thickness of the second protecting film 114 is greater than 2000 nm, the second protecting film 114 is easily cracked due to the thermal shock and loses the protecting function due to excessively great stress.

In an embodiment, the material of the second protecting film 114 is conductive ceramic or metal. Compared with the first protecting film 113 which is made of a non-conductive material, the second protecting film 114 is made of a conductive material, so that the second protecting film 114 does not affect the electrical connection between the connection portion 1122 and the power supply assembly 2 while protecting the connection portion 1122 from the corrosion of the aerosol-generating substance. Since the material of the heating material layer 112 is metal, the thermal expansion coefficient of the conductive ceramic or metal matches the thermal expansion coefficient of the metal heating material layer 112, and the adhesion force of the conductive ceramic or metal matches the adhesion force of the metal heating material layer 112, such that the second protecting film 114 may be difficult to fall off the connection portion 1122 and thus achieve a better protecting effect when the conductive ceramic or metal is served as the second protecting film 114. The conductive ceramic or metal is served as the second protecting film 114, such that the contacting resistance is reduced.

When the material of the second protecting film 114 is the conductive ceramic, the material of the conductive ceramic includes one or more of titanium nitride or titanium diboride. It may be understood that, the conductive ceramic is more resistant to the corrosion of the aerosol-generating substance than the metal.

It should be noted that, the connection portion 1122 of the heating material layer 112 and the second protecting film 114 form an electrode. The second protecting film 114 is arranged on the connection portion 1122, which reduces the resistance and may be served as the electrode. The thickness of the heating portion 1121 and the thickness of the connection portion 1122 may be the same or may be different. To reduce a resistance value of the connection portion 1122, the thickness of the connection portion 1122 may also be greater than that of the heating portion 1121.

As shown in FIG. 3a and FIG. 4, in this embodiment, the liquid-guiding substrate 111 is a dense liquid-guiding substrate and includes a plurality of first micropores 1113. The plurality of first micropores 1113 are designed through holes penetrating the first surface 1111 and the second surface 1112. The aerosol-generating substance in the liquid storage cavity 14 reaches the liquid-guiding substrate 111 of the heating body 11 through the liquid supplying channel 1211. The aerosol-generating substance is guided from the second surface 1112 of the liquid-guiding substrate 111 to the first surface 1111 of the liquid-guiding substrate 111 through the capillary force of the plurality of first micropores 1113 of the liquid-guiding substrate 111, so that the aerosol-generating substance is atomized by the heating material layer 112 arranged on the first surface 1111. That is, the plurality of first micropores 1113 are in fluid communication with the liquid storage cavity 14 through the liquid supplying channel 1211. The material of the liquid-guiding substrate 111 may be quartz, glass, or dense ceramic, and in this case, the plurality of first micropores 1113 are straight through holes. When the material of the liquid-guiding substrate 111 is glass, the glass may be one of common glass, quartz glass, borosilicate glass, or photosensitive lithium aluminosilicate glass.

In an embodiment, the plurality of first micropores 1113 are only provided in the heating region a of the liquid-guiding substrate 111, and no first micropore 1113 is provided in the electrode region b. In an embodiment, the heating portion 1121 is not only arranged on the first surface 1111, but is also arranged on the inner surface of each of the plurality of first micropores 1113. The first protecting film 113 is also arranged in each of the plurality of first micropores 1113 and totally covers the heating portion 1121 arranged on the inner surface of each of the plurality of first micropores 1113.

It may be understood that, when the power of the electronic atomization device ranges from 6 W to 8.5 W and the voltage of the battery ranges from 2.5 V to 4.4 V, to reach an operating resistance of the battery, the resistance of the heating material layer 112 of the heating body 11 at the normal temperature ranges from 0.5 S2 to 2 Ω. In this embodiment, the heating material layer 112 covers the entire heating region a.

In the present disclosure, the plurality of first micropores 1113 having the capillary forces are provided on the liquid-guiding substrate 111, so that a porosity of the heating body 11 may be accurately controlled, thereby improving the product consistency. That is, in the mass production, the porosity of the liquid-guiding substrate 111 in the heating body 11 is subsequently consistent, and the thickness of the heating material layer 112 formed on the liquid-guiding substrate 111 is uniform, so that atomization effects of electronic atomization devices produced in the same batch are consistent.

Compared with an existing cotton core heating body and a porous ceramic heating body, the heating body 11 provided in the present disclosure which has a thin-sheet structure and is defined with the first micropores 1113 has a shorter liquid supplying channel and a greater liquid supplying speed, but also has a larger risk of liquid leakage. Therefore, the inventor of the present disclosure researched an impact of a ratio of the thickness of the liquid-guiding substrate 111 to a pore size of the first micropore 1113 on the liquid supplying of the heating body 11, and found that the risk of liquid leakage may be reduced by increasing the thickness of the liquid-guiding substrate 111 and reducing the pore size of the first micropore 1113 while a liquid supplying speed may also be reduced; and the liquid supplying speed may be increased by reducing the thickness of the liquid-guiding substrate 111 and increasing the pore size of the plurality of first micropore 1113 while the risk of liquid leakage may also be increased. That is, the risk of liquid leakage conflicts with the liquid supplying speed. Therefore, in the present disclosure, the thickness of the liquid-guiding substrate 111, the pore size of the first micropore 1113, and the ratio of the thickness of the liquid-guiding substrate 111 to the pore size of the first micropore 1113 are designed. The heating body 11 is configured to operate at the power ranging from 6 W to 8.5 W, and the voltage ranging from 2.5 V to 4.4 V, such that sufficient liquid supplying is achieved while the liquid leakage is prevented V. The thickness of the liquid-guiding substrate 111 is a distance between the first surface 1111 and the second surface 1112.

In addition, the inventor of the present disclosure further researched a ratio of a distance between centers of two adjacent first micropores 1113 to the pore size of the first micropore 1113, and found that when the ratio of the distance between the centers of the two adjacent first micropores 1113 to the pore size of the first micropore 1113 is excessively great, the liquid-guiding substrate 111 has a greater intensity and is easy to be manufactured, while the porosity is excessively less, which easily leads to an insufficient liquid supplying amount. When the ratio of the distance between the centers of the adjacent two first micropores 1113 to the pore size of the first micropore 1113 is excessively small, the porosity is greater, which brings a sufficient liquid supplying amount, while the liquid-guiding substrate 111 has less intensity and is hard to be manufactured. Therefore, in the present disclosure, the ratio of the distance between the centers of the adjacent two first micropores 1113 to the pore size of the first micropore 1113 is further designed, so that the intensity of the liquid-guiding substrate 111 is improved as much as possible in a condition that the liquid supplying capability is met.

A case in which the material of the liquid-guiding substrate 111 is glass is described below.

Both the first surface 1111 and the second surface 1112 include smooth surfaces, and the first surface 1111 is a flat surface. That is, the first surface 1111 of the liquid-guiding substrate 111 is a smooth flat surface. The first surface 1111 is configured to be the smooth flat surface, such that a metal material can deposited to form a film with a less thickness, i.e., the heating material layer 112 is formed on the first surface 1111 of the liquid-guiding substrate 111.

In an embodiment, both the first surface 1111 and the second surface 1112 of the liquid-guiding substrate 111 are smooth flat surfaces, and the first surface 1111 and the second surface 1112 of the liquid-guiding substrate 111 are arranged parallel to each other. The axi of the first micropores 1113 is perpendicular to the first surface 1111 and the second surface 1112, and in this case, the thickness of the liquid-guiding substrate 111 is equal to the length of the first micropore 1113. It may be understood that, the second surface 1112 is parallel to the first surface 1111, and the plurality of first micropores 1113 penetrate from the first surface 1111 to the second surface 1112, so that a manufacture process of the liquid-guiding substrate 111 is simple and the cost is reduced. The distance between the first surface 1111 and the second surface 1112 is the thickness of the liquid-guiding substrate 111.

In an embodiment, the first surface 1111 of the liquid-guiding substrate 111 is the smooth flat surface, and the second surface 1112 of the liquid-guiding substrate 111 is a smooth non-flat surface such as an inclined surface, a cambered surface, a serrated surface, or the like. The second surface 1112 may be designed according to the specific requirement, in a condition that the plurality of first micropores 1113 penetrate the first surface 1111 and the second surface 1112.

In an embodiment, a cross section of the first micropore 1113 is in shape of a circle. The plurality of first micropores 1113 may be straight through holes having uniform pore sizes or may be straight through holes having non-uniform pore sizes, in a condition that a changing range of the pore size falls within 50%. For example, due to the limitation of a preparation process, the first micropore 1113 provided on the glass through laser induction and corrosion, may generally have greater pore sizes at two ends and a less pore size at a middle portion. Therefore, it is only required to ensure that the pore size at the middle portion of the first micropore 1113 is greater than or equal to a half of the pore sizes at the two ends.

The thickness of the liquid-guiding substrate 111, the pore size of the first micropore 1113, the ratio of the thickness of the liquid-guiding substrate 111 to the pore size of the first micropore 1113, and the ratio of the distance between the centers of the adjacent two first micropores 1113 to the pore size of the first micropore 1113 in a case where the material of the liquid-guiding substrate 111 is the glass, and both the first surface 1111 and the second surface 1112 of the liquid-guiding substrate 111 are the smooth flat surfaces and are arranged parallel to each other, are described below.

The thickness of the liquid-guiding substrate 111 ranges from 0.1 mm to 1 mm. When the thickness of the liquid-guiding substrate 111 is greater than 1 mm, a liquid supplying requirement cannot be met, leading to a decrease in the amount of the aerosol, a great heat loss, and a high cost for providing the plurality of first micropores 1113. When the thickness of the liquid-guiding substrate 111 is less than 0.1 mm, the intensity of the liquid-guiding substrate 111 cannot be ensured, which affects the improvement of the performance of the electronic atomization device. In an embodiment, the thickness of the liquid-guiding substrate 111 ranges from 0.2 mm to 0.5 mm. It may be understood that, the thickness of the liquid-guiding substrate 111 is selected according to an actual requirement.

The pore size of the first micropore 1113 on the liquid-guiding substrate 111 ranges from 1 µm to 100 µm. When the pore size of the first micropore 1113 is less than 1 µm, the liquid supplying requirement cannot be met, leading to the decrease in the amount of the aerosol. When the pore size of the first micropore 1113 is greater than 100 µm, the aerosol-generating substance may easily leak out from the plurality of first micropores 1113 to the first surface 1111 to cause the liquid leakage, leading to a decrease in the atomization efficiency. In an embodiment, the pore size of the first micropore 1113 ranges from 20 µm to 50 µm. It may be understood that, the pore size of the first micropore 1113 is selected according to the actual requirement.

In an embodiment, the ratio of the thickness of the liquid-guiding substrate 111 to the pore size of the first micropore 1113 ranges from 20:1 to 3:1. In an embodiment, the ratio of the thickness of the liquid-guiding substrate 111 to the pore size of the first micropore 1113 ranges from 15:1 to 5:1. When the ratio of the thickness of the liquid-guiding substrate 111 to the pore size of the first micropore 1113 is greater than 20:1, the amount of the aerosol-generating substance provided to the heating body 11 by means of the capillary force of the plurality of first micropores 1113 may be difficult to meet an atomization amount required by the heating body 11, which not only easily leads to dry burning but also causes a decrease in the amount of the aerosol generated in a single atomization process. When the ratio of the thickness of the liquid-guiding substrate 111 to the pore size of the first micropore 1113 is less than 3:1, the aerosol-generating substance may easily leak out from e the plurality of first micropores 1113 to the first surface 1111 to cause a waste of the aerosol-generating substance, leading to the decrease in the atomization efficiency and further causing a decrease in a total amount of the aerosol.

The ratio of the distance between the centers of the two adjacent first micropores 1113 to the pore size of the first micropore 1113 ranges from 3:1 to 1.5:1, so that the intensity of the liquid-guiding substrate 111 is improved as much as possible in a condition that the plurality of first micropores 1113 on the liquid-guiding substrate 111 meet the liquid supplying capability. In an embodiment, the ratio of the distance between the centers of the two adjacent first micropores 1113 to the pore size of the first micropore 1113 ranges from 3:1 to 2:1. In an embodiment, the ratio of the distance between the centers of the two adjacent first micropores 1113 to the pore size of the first micropore 1113 ranges from 3:1 to 2.5:1.

In a specific embodiment, the ratio of the thickness of the liquid-guiding substrate 111 to the pore size of the first micropore 1113 ranges from 15:1 to 5:1, and the ratio of the distance between the centers of the two adjacent first micropores 1113 to the pore size of the first micropore 1113 ranges from 3:1 to 2.5:1.

In this embodiment, the liquid-guiding substrate 111 is in the shape of a flat plate. For example, the liquid-guiding substrate 111 is in the shape of a rectangular plate or a circular plate, which is designed as required. In some other embodiments, the liquid-guiding substrate 111 is in the shape of an arc or a barrel. The plurality of first micropores 1113 are arranged in an array in the heating region a. That is, the plurality of first micropores 1113 provided on the liquid-guiding substrate 111 are regularly arranged, and distances between centers of adjacent first micropores 1113 among the plurality of first micropores 1113 are the same. The pore sizes of the plurality of first micropores 1113 may be the same or may be different, which is designed as required.

The liquid-guiding substrate 111 in the heating body 11 is a dense material, so that the liquid-guiding substrate may have the structure supporting function. Compared with a spring-shaped metal heating wire in the existing cotton core heating body or a metal thick-film wire in the existing porous ceramic heating body, both the intensity and the thickness of the heating material layer 112 in the heating body 11 are not required, and the heating material layer 112 may adopt a metal material having a low resistivity, such as gold, or aluminum.

In an embodiment, the heating material layer 112 formed on the first surface 1111 of the liquid-guiding substrate 111 is a heating film. The thickness of the heating material layer 112 ranges from 200 nm to 5 µm, i.e., the heating material layer 112 has a less thickness. In an embodiment, the thickness of the heating material layer 112 ranges from 200 nm to 1 µm. In an embodiment, the thickness of the heating material layer 112 ranges from 200 nm to 500 nm. When the heating material layer 112 is the heating film, a plurality of second micropores 1123 corresponding to the plurality of first micropores 1113 are provided on the heating material layer 112. The heating material layer 112 is further formed on the inner surface of each of the plurality of first micropores 1113. In an embodiment, the heating material layer 112 is further formed on the entire inner surface of each of the plurality of first micropores 1113. The heating material layer 112 is arranged on the inner surface of each of the plurality of first micropores 1113, so that the aerosol-generating substance may be atomized in the plurality of first micropores 1113, and thereby the atomization effect is improved.

It may be understood that, when the thickness of the heating material layer 112 is greater than 5 µm, the heating material layer 112 is generally formed in a printing manner, and the plurality of first micropores 1113 may be blocked when the thickness of the heating material layer 112 is excessively great. The thickness of the heating material layer 112 may range from 5 µm to 100 µm. In this embodiment, the heating material layer 112 covers the entire heating region a, so as to prevent the liquid supplying from being affected. The thickness of the heating material layer 112 is less than or equal to 5 µm.

In an embodiment, the resistivity of the heating material layer 112 is less than or equal to 0.06*10⁻⁶ S2 . m. On the basis that the resistance of the heating material layer 112 at the normal temperature ranges from 0.5 Ω to 2 Ω, the metal material having a low electrical conductivity is adopted in the present disclosure to form a thinner metal film, so as to reduce an influence on the pore size of the first micropore 1113 as much as possible. The thinner the heating material layer 112 is, the less the influence on the pore size of the first micropore 1113 is, and a better atomization effect may be achieved. In addition, a thinner heating material layer 112 brings a small amount of the heat absorbed by the heating material layer 112, a lower electric and heat loss, and a great temperature increasing speed of the heating body 11.

In an embodiment, the metal material of the heating material layer 112 includes silver and alloy thereof, copper and alloy thereof, aluminum and alloy thereof, gold and alloy thereof, nickel and alloy thereof, chromium and alloy thereof, platinum and alloy thereof, titanium and alloy thereof, zirconium and alloy thereof, palladium and alloy thereof, or iron and alloy thereof. In an embodiment, the material of the heating material layer 112 may include the aluminum and the alloy thereof and the gold and the alloy thereof. Since the liquid aerosol-generating substance includes various flavors, fragrances, additives, and an element such as sulphur, phosphorus, or chlorine. The gold includes quite strong chemical inertness, and a dense oxide thin film may be generated on the surface of the aluminum, so that the two materials are quite stable in the liquid aerosol-generating substance, and are selected as the material of the heating material layer 112.

In an embodiment, the heating material layer 112, the first protecting film 113, and the second protecting film 114 may be formed on the first surface 1111 of the liquid-guiding substrate 111 by means of a physical vapor deposition (such as magnetron sputtering, vacuum evaporation, or ion plating) or a chemical vapor deposition (ion-assisted chemical deposition, laser-assisted chemical deposition, or metal organic compound deposition).

It may be understood that, by means of forming processes of the heating material layer 112 and the first protecting film 113, the plurality of first micropores 1113 may not be covered by the heating material layer and the first protecting film. Both the heating material layer 112 and the first protecting film 113 extend into the wall surface of each of the plurality of first micropores 1113. While the heating material layer 112 and the first protecting film 113 are formed on the first surface 1111 of the liquid-guiding substrate 111 by means of the physical vapor deposition or the chemical vapor deposition, while the heating material layer 112 and the first protecting film 113 are also formed on the inner surface of each of the plurality of first micropores 1113. When the heating material layer 112 and the first protecting film 113 are formed on the first surface 1111 of the liquid-guiding substrate 111 by means of the magnetron sputtering, a metal atom is perpendicular to the first surface 1111 and parallel to the inner surface of each of the plurality of first micropores 1113, so that the metal atom is more easily deposited on the first surface 1111.When the thickness of the heating material layer 112 and the first protecting film 113 formed by depositing metal atoms on the first surface 1111 is 1 µm, while the thickness of the metal atoms deposited on the inner surface of each of the plurality of first micropores 1113 is far less than 1 µm and even less than 0.5 µm. A less the thickness of the heating material layer 112 and the first protecting film 113 deposited on the first surface 1111 is, the less the thickness of the heating material layer 112 and the first protecting film 113 formed on the inner surface of each of the plurality of first micropores 1113, and the less the influence on the pore sizes of the plurality of first micropores 1113 are. Since the thicknesses of the heating material layer 112 and the first protecting film 113 are far less than the pore size of the first micropore 1113, and the thickness of the portions of the heating material layer 112 and the first protecting film 113 deposited in each of the plurality of first micropores 1113 is less than the thickness of the portions deposited on the first surface 1111 of the liquid-guiding substrate 111, the influence of the heating material layer 112 and the first protecting film 113 deposited in each of the plurality of first micropores 1113 on the pore sizes of the plurality of first micropores 1113 may be omitted.

In some other embodiments, the material of the liquid-guiding substrate 111 is porous ceramic, and a plurality of capillary holes interconnected and distributed in an undersigned manner are provided in the porous ceramic. The capillary holes of the porous ceramic are configured to guide liquid. The liquid-guiding substrate 111 includes a plurality of undesigned through holes. The first protecting film 113 is arranged on the heating portion 1121 of the heating material layer 112, and the second protecting film 114 is arranged on the connection portion 1122 of the heating material layer 112, so as to protect the heating material layer 112. That is, the first protecting film 113 and the second protecting film 114 provided in the present disclosure may be applied to the surface of a conventional porous ceramic heating body to protect its heating material layer.

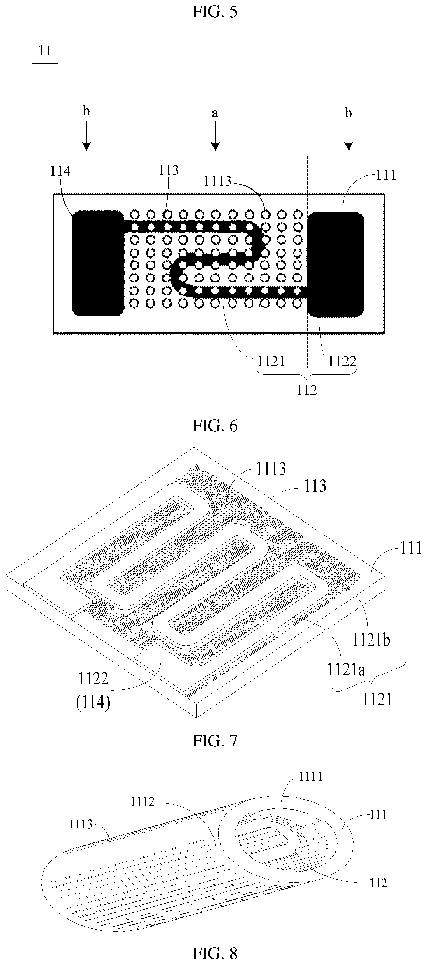

As shown in FIG. 5, FIG. 5 is a structural schematic view of the heating body according to a second embodiment of the present disclosure. The liquid-guiding substrate 111 may also be composite ceramic. The liquid-guiding substrate 111 includes a porous ceramic layer and a dense ceramic layer arranged in a stacking manner. The dense ceramic layer includes a plurality of designed straight through holes perpendicular to the direction of the thickness of the liquid-guiding substrate 111. The heating material layer 112 is arranged on the surface of the dense ceramic layer away from the porous ceramic layer. The liquid-guiding substrate 111 includes a first sub liquid-guiding substrate 111a and a second sub liquid-guiding substrate 111b, that is, the first sub liquid-guiding substrate 111a is the porous ceramic layer, and the second sub liquid-guiding substrate 111b is the dense ceramic layer. The surface of the first sub liquid-guiding substrate 111a away from the second liquid-guiding substrate 111b is the second surface 1112 of the liquid-guiding substrate 111, and the surface of the second sub liquid-guiding substrate 111b away from the first liquid-guiding substrate 111a is the first surface 1111. The material of the first sub liquid-guiding substrate 111a is the porous ceramic, and the first sub liquid-guiding substrate 111a includes a plurality of undesigned through holes. The material of the second sub liquid-guiding substrate 111b is the dense ceramic, and the second sub liquid-guiding substrate 111b includes the plurality of first micropores 1113. The plurality of first micropores 1113 are through holes, and an axis of each of the plurality of first micropores 1113 is parallel to the direction of the thickness of the second sub liquid-guiding substrate 111b. The heating material layer 112 is arranged on the surface of the second sub liquid-guiding substrate 111b away from the first liquid-guiding substrate 111a. The first protecting film 113 is arranged on the heating portion 1121 of the heating material layer 112, and the second protecting film 114 is arranged on the connection portion 1122 of the heating material layer 112, so that the heating material layer 112 is protected.

As shown in FIG. 6, FIG. 6 is a structural schematic view of the heating body according to a third embodiment of the present disclosure.

A difference between the heating body 11 shown in FIG. 6 and the heating body 11 shown in FIG. 3a lies in that: in FIG. 3a, the heating material layer 112 covers the entire heating region a or crosses the entire heating region a., while in FIG. 6, the heating material layer 112 covers a part of the heating region a. That is, the shape of the heating material layer 112 in FIG. 6 is different from the shape of the heating material layer 112 in FIG. 3a, and other same structures are not repeated herein.

As shown in FIG. 6, the heating portion 1121 of the heating material layer 112 is in the shape of a S-shaped bended strip, to form a temperature field having a temperature gradient on the first surface 1111 of the liquid-guiding substrate 111. That is, a high-temperature region and a low-temperature region are formed on the first surface 1111 of the liquid-guiding substrate 111, so as to atomize various components in the aerosol-generating substance to the greatest extent. Two ends of the heating portion 1121 are connected to a connection portion 1122, respectively. The sizes of the connection portions 1122 are greater than the size of the heating portion 1121, such that the connection portion 1122 may be better electrically connected to the power supply assembly 2. The resistivity of the heating material layer 112 is less than or equal to 0.06* 10-6 Ω·m. In an embodiment, the heating portion 1121 and the connection portion 1122 are integrally formed.

The first protecting film 113 is arranged on the surface of the heating portion 1121 away from the liquid-guiding substrate 111, the second protecting film 114 is arranged on the surface of the connection portion 1122 away from the liquid-guiding substrate 111. Reference may be made to the foregoing description of the first protecting film 113 and the second protecting film 114 for details.

In an embodiment, the heating material layer 112 formed on the first surface 1111 of the liquid-guiding substrate 111 is the heating film, and the thickness of the heating material layer 112 ranges from 200 nm to 5 µm. That is, the thickness of the heating material layer 112 is less. In an embodiment, the thickness of the heating material layer 112 ranges from 200 nm to 1 µm. In an embodiment, the thickness of the heating material layer 112 ranges from 200 nm to 500 nm. In an embodiment, the heating material layer 112 is formed by means of the physical vapor deposition (such as the magnetron sputtering, the vacuum evaporation, or the ion plating) or the chemical vapor deposition (the iron-assisted chemical deposition, the laser-assisted chemical deposition, or the metal organic compound deposition).

In another embodiment, the thickness of the heating material layer 112 formed on the first surface 1111 of the liquid-guiding substrate 111 ranges from 5 µm to 100 µm. That is, the thickness of the heating material layer 112 is greater. In an embodiment, the thickness of the heating material layer 112 ranges from 5 µm to 50 µm. In an embodiment, the heating material layer 112 is formed on the first surface 1111 of the liquid-guiding substrate 111 in the printing manner. That is, the heating material layer 112 is a printed metal slurry layer. Since the first surface 1111 of the liquid-guiding substrate 111 has a low roughness, a consecutive film shape may be formed when the thickness of the heating material layer 112 is less than 100 µm.

It may be understood that, the heating material layer 112 in FIG. 6 covers a part of the heating region a, and the thickness of the heating material layer 112 may be set to range from 5 µm to 100 µm. Even if a region where the heating material layer 112 is arranged blocks a part of the plurality of first micropores 1113, the other first micropores 1113 may be configured to supply the liquid. The liquid-guiding substrate 111 of the heating body 11 shown in FIG. 6 may be the dense liquid-guiding substrate, the porous ceramic, or the composite ceramic (as the liquid-guiding substrate 111 shown in FIG. 5).

As shown in FIG. 7, FIG. 7 is a structural schematic view of the heating body according to a fourth embodiment of the present disclosure.

A difference between the heating body 11 shown in FIG. 7 and the heating body 11 shown in FIG. 3a lies in that: the shape of the heating material layer 112 of the heating body 11 shown in FIG. 7 is different from the shape of the heating material layer 112 of the heating body 11 shown in FIG. 3a, and other same structures are not repeated herein again.

As shown in FIG. 7, the liquid-guiding substrate 111 is in the shape of the flat plate. The heating portion 1121 of the heating material layer 112 includes a plurality of first sub heating portions 1121a extending in a first direction and a plurality of second sub heating portions 1121b extending in a second direction. Two adjacent first sub heating portions 1121a are connected by one of the second heating portions 1121b. Two connection portions 1122 are arranged on the same side of the heating portion 1121. The width of each of the connection portion 1122 is greater than the width of the heating portion 1121.

The first protecting film 113 is arranged on the surface of the heating portion 1121 away from the liquid-guiding substrate 111, and the second protecting film 114 is arranged on the surface of the connection portion 1122 away from the liquid-guiding substrate 111. Reference may be made to the foregoing descriptions of the first protecting film 113 and the second protecting film 114 for details.

As shown in FIG. 8, FIG. 8 is a structural schematic view of the heating body according to a fifth embodiment of the present disclosure.

A difference between the heating body 11 shown in FIG. 8 and the heating body 11 shown in FIG. 3a lies in that: the shape of the heating body 11 in FIG. 8 is different from the shape of the heating body 11 in FIG. 3a. Other same structures are not repeated herein.

As shown in FIG. 8, the liquid-guiding substrate 111 is in the shape of the barrel. The liquid-guiding substrate 111 is the dense liquid-guiding substrate, and includes the plurality of first micropores 1113. The plurality of first micropores 1113 are straight through holes penetrating the first surface 1111 and the second surface 1112. The first surface 1111 is the inner surface of the barrel-shaped liquid-guiding substrate 111, and the second surface 1112 is an outer surface of the barrel-shaped liquid-guiding substrate 111. The heating material layer 112 is arranged on the first surface 1111 of the liquid-guiding substrate 111. The first protecting film 113 and the second protecting film 114 are arranged on the surface of the heating material layer 112 away from the liquid-guiding substrate 111. It should be noted that, the first protecting film 113 and the second protecting film 114 are not marked in FIG. 8.

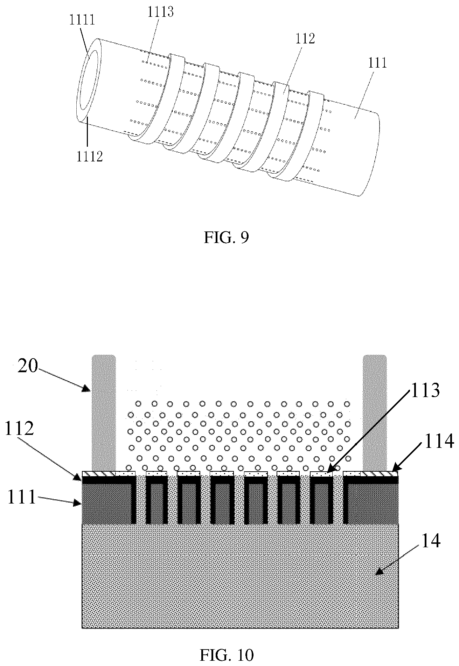

As shown in FIG. 9, FIG. 9 is a structural schematic view of the heating body according to a sixth embodiment of the present disclosure.

A difference between the heating body 11 shown in FIG. 9 and the heating body 11 shown in FIG. 3a lies in that: the shape of the heating body 11 in FIG. 9 is different from the shape of the heating body 11 in FIG. 3a. Other same structures are not repeated herein.

As shown in FIG. 9, the liquid-guiding substrate 111 is in the shape of the barrel. The liquid-guiding substrate 111 is the dense liquid-guiding substrate, and includes the plurality of first micropores 1113. The plurality of first micropores 1113 are straight through holes penetrating the first surface 1111 and the second surface 1112. The first surface 1111 is the inner surface of the barrel-shaped liquid-guiding substrate 111, and the second surface 1112 is the outer surface of the barrel-shaped liquid-guiding substrate 111. The heating material layer 112 is arranged on the second surface 1112 of the liquid-guiding substrate 111. The first protecting film 113 and the second protecting film 114 are arranged on the surface of the heating material layer 112 away from the liquid-guiding substrate 111. It should be noted that, the first protecting film 113 and the second protecting film 114 are not marked in FIG. 9.

A relationship among the material of the heating material layer 112, the material of the first protecting film 113, and the material of the second protecting film 114 and the service life of the heating body 11, and a relationship among the material of the first protecting film 113, and the material of the second protecting film 114 and the atomization amount will be verified through experiments below. As shown in FIG. 10, FIG. 10 is a schematic view of a process of a wet burning experiment on the heating body according to the present disclosure.

Experiment one: the heating body 11 is loaded with a cartridge and wet burnt to evaluate the service life of the heating body 11. Experiment conditions: the power is supplied at a constant power of 6.5 W, 3s of inhalation and stopped for 27s, and the aerosol-generating substance is cola ice, 30 mg. A case in which the heating body 11 is provided with the first protecting film 113 is compared to a case in which the heating body 11 is not provided with the protecting film. The first protecting film 113 is configured to include different materials for comparison. A normal applied environment of the electronic atomization device is simulated to perform the experiments (As shown in FIG. 10). A comparison result is shown in Table 3, and the relationship among the material of the heating material layer 112, and the material of the first protecting film 113, and the service life of the heating body 11 is obtained. In FIG. 10, the power is supplied by means of a direct current power supply. An ejector pin 20 of the power supply assembly 2 (the ejector pin 20 is electrically connected to a battery) is connected to a corresponding connection portion 1122 of the heating material layer 112 to control the energized power and the energized duration.

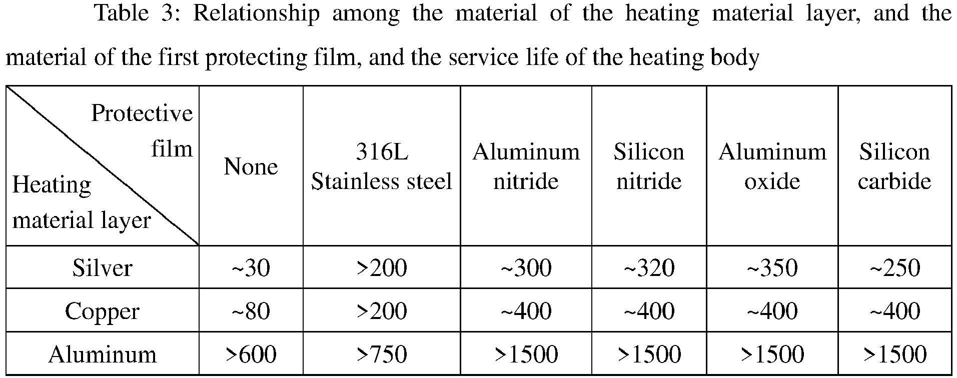

The flavors and fragrances and the additives include the element such as the sulphur, the phosphorus, or the chlorine, when the heating body 11 is not provided with the first protecting film 113, the silver and the copper are easily corroded by the flavors and fragrances and the additives in the aerosol-generating substance when serving as the material of the heating material layer 112.The requirement of the service life is difficult to be met. When serving as the material of the heating material layer 112, the aluminum may support over 600 times of thermal cycles, which meets a serving condition of the closed electronic atomization device, but is difficult to meet a requirement of an open electronic atomization device being sucked for 1500 times.

Therefore, the first protecting film 113 is arranged on the surface of the heating material layer 112 to improve the service life of the heating material layer 112. The material of the first protecting film 113 is a ceramic material resistant to the corrosion of the aerosol-generating substance, such as the aluminum nitride, the silicon nitride, the aluminum oxide, the silicon oxide, the silicon carbide, or the zirconium oxide. No matter the material of the heating material layer 112 is the silver, the copper, or the aluminum, the service life of the heating body 11 may all be greatly improved after the first protecting film 113 is adopted.

Experiment two: the heating body 11 is loaded with the cartridge and wet burnt to evaluate the service life of the heating body 11. Experiment conditions: the power is supplied at the constant power of 6.5 W, 3s of inhalation and stopped for 27 seconds, and the aerosol-generating substance is the cola ice, 30 mg. Atomization amounts of the heating body 11 corresponding to first protecting films 113 including different materials are compared. The normal serving environment of the electronic atomization device is simulated to perform the experiments (As shown in FIG. 10). A comparison result is shown in Table 4, and the relationship between the material of the first protecting film 113 and the atomization amount is obtained. In FIG. 10, the power is supplied by the direct current power supply. The ejector pin 20 of the power supply assembly 2 (the ejector pin 20 is electrically connected to the battery) is connected to the corresponding connection portion 1122 of the heating material layer 112 to control the energized power and the energized duration.

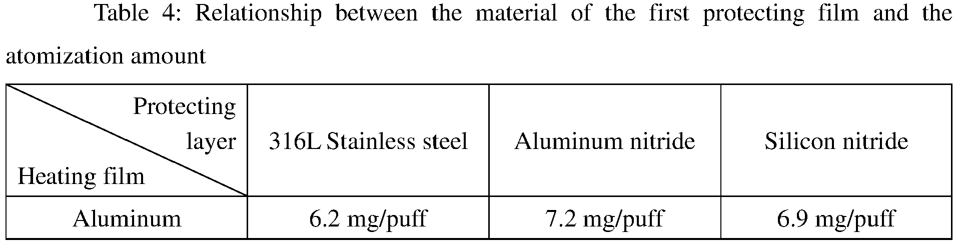

It can be read from FIG. 4, the atomization amount is apparently improved when the material of the first protecting film 113 selects the ceramic material (such as the aluminum nitride or the silicon nitride) when compared with the metal material (such as the 316L stainless steel).

The above descriptions are merely some embodiments of the present disclosure, and the scope of the present disclosure is not limited thereto. All equivalent structure or process changes made according to the content of this specification and the accompanying drawings in the present disclosure, or direct or indirect applications in other related technical fields shall fall within the scope of the present disclosure.

## Claims

1. A heating body (11), applied to an electronic atomization device (100) and configured to atomize an aerosol-generating substance, the heating body (11) comprises:
a liquid-guiding substrate (111);
a heating material layer (112), arranged on a first surface (1111) of the liquid-guiding substrate (111), wherein the heating material layer (112) is a resistance heating material, and comprises:
a heating portion (1121), wherein a portion of the liquid-guiding substrate (111), where the heating portion (1121) is arranged, is defined as a heating region (a); and
a connection portion (1122), wherein another portion of the liquid-guiding substrate (111), where the connection portion (1122) is arranged, is defined as an electrode region (b) **characterized by**
a first protecting film (113), at least partially arranged on the surface of the heating portion (1121) away from the liquid-guiding substrate (111), wherein the material of the first protecting film (113) is non-conductive and resistant to corrosion of the aerosol-generating substance; and
a second protecting film (114), at least partially arranged on the surface of the connection portion (1122) away from the liquid-guiding substrate (111), wherein the material of the second protecting film (114) is conductive and resistant to the corrosion of the aerosol-generating substance.

2. The heating body (11) according to claim 1, wherein the material of the first protecting film (113) is ceramic or glass;
wherein when the material of the first protecting film (113) is the ceramic, the material of the ceramic comprises one or more of aluminum nitride, silicon nitride, aluminum oxide, silicon oxide, silicon carbide, and zirconium oxide.

3. The heating body (11) according to claim 1, wherein the thickness of the first protecting film (113) ranges from 10 nm to 1000 nm.

4. The heating body (11) according to claim 1, wherein the material of the second protecting film (114) is conductive ceramic or metal;
wherein when the material of the second protecting film (114) is the conductive ceramic, the material of the conductive ceramic comprises one or more of titanium nitride and titanium diboride.

5. The heating body (11) according to claim 1, wherein the thickness of the second protecting film (114) ranges from 10 nm to 2000 nm.

6. The heating body (11) according to claim 1, wherein the liquid-guiding substrate (111) is a dense liquid-guiding substrate (111), and the liquid-guiding substrate (111) further comprises a second surface (1112) opposite to the first surface (1111), the liquid-guiding substrate (111) comprises a plurality of first micropores (1113), and the plurality of first micropores (1113) are designed through holes penetrating the first surface (1111) and the second surface (1112);
wherein the material of the liquid-guiding substrate (111) comprises quartz, glass, or dense ceramic, and the plurality of first micropores (1113) are straight through holes;
wherein the heating material layer (112) and the first protecting film (113) extend into the wall surface of each of the plurality of first micropores (1113).

7. The heating body (11) according to claim 1, wherein the material of the liquid-guiding substrate (111) is porous ceramic, and the liquid-guiding substrate (111) comprises a plurality of undesigned through holes; or
the liquid-guiding substrate (111) comprises a porous ceramic layer and a dense ceramic layer arranged in a stacking manner, the dense ceramic layer comprises a plurality of designed straight through holes perpendicular to the direction of the thickness of the liquid-guiding substrate (111), and the heating material layer (112) is arranged on the surface of the dense ceramic layer away from the porous ceramic layer.

8. The heating body (11) according to claim 1, wherein the heating material layer (112) is a heating film, and the thickness of the heating film ranges from 200 nm to 5 µm.

9. The heating body (11) according to claim 1, wherein the thickness of the heating material layer (112) ranges from 5 µm to 100 µm, and the heating material layer (112) is a printed metal slurry layer.

10. The heating body (11) according to claim 1, wherein the first protecting film (113) covers the entire heating portion (1121), and the second protecting film (114) covers the entire connection portion (1122).

11. The heating body (11) according to claim 1, wherein the liquid-guiding substrate (111) is in the shape of a cylinder, the liquid-guiding substrate (111) comprises an inner surface and an outer surface, and the heating material layer (112) is arranged on the inner surface or the outer surface.

12. The heating body (11) according to claim 1, wherein the heating material layer (112), the first protecting film (113), and the second protecting film (114) are formed on the first surface (1111) of the liquid-guiding substrate (111).

13. The heating body (11) according to claim 1, wherein the connection portion (1122) of the heating material layer (112) and the second protecting film (114) form an electrode.

14. An atomization assembly (1), **characterized by** comprising:
a liquid storage cavity (14), configured to store a liquid aerosol-generating substance; and
a heating body (11) according to any one of claims 1 to 13, and the heating body (11) is in fluid communication with the liquid storage cavity (14).

15. An electronic atomization device (100), **characterized by** comprising:
an atomization assembly (1) according to claim 14; and
a power supply assembly (2), electrically connected to the heating body (11).

## Patentansprüche

1. Ein Heizkörper (11) für die Anwendung an einer elektronischen Zerstäubungsvorrichtung (100), der konfiguriert ist, um eine Aerosol erzeugende Substanz zu zerstäuben, wobei der Heizkörper (11) Folgendes umfasst:
Ein flüssigkeitsleitendendes Substrat (111);
eine Heizmaterialschicht (112), die an einer ersten Oberfläche (1111) des flüssigkeitsleitenden Substrats (111) angeordnet ist, wobei die Heizmaterialschicht (112) ein Widerstandsheizmaterial ist, und Folgendes umfasst:
einen Heizteil (1121), wobei ein Teil des flüssigkeitsleitenden Substrats (111), an dem der Heizteil (1121) angeordnet ist, als ein Heizbereich (a) definiert wird; und
ein Anschlussteil (1122), bei dem ein anderer Teil des flüssigkeitsleitentenden Substrats (111), an dem der Anschlussteil (1122) angeordnet ist, als ein Elektrodenbereich (b) definiert wird;
**gekennzeichnet durch**
eine erste Schutzfolie (113), die mindestens teilweise auf der Oberfläche des Heizteils (1121) angeordnet ist, die abgewandt ist vom flüssigkeitsleitenden Substrat (111), wobei das Material der ersten Schutzfolie (113) nicht leitend ist und korrosionswiderstandfähig gegenüber der Aersol erzeugenden Substanz; und
eine zweite Schutzfolie (114), die mindestens teilweise auf der Oberfläche des Anschlussteils (1122) abgewandt vom flüssigkeitsleitenden Substrat (111) angeordnet ist, wobei das Material der zweiten Schutzfolie (114) leitfähig ist und korrosionswiderstandfähig gegenüber der Aerosol erzeugenden Substanz.

2. Der Heizkörper (11) gemäss Anspruch 1, bei dem das Material der ersten Schutzfolie(113) Keramik oder Glas ist;
wobei, wenn das Material der ersten Schutzfolie (113) Keramik ist, die Keramik eins oder meherere folgender Materialien umfasst: Aluminiumnitrid, Siliziumnitrid, Aluminiumoxid, Siliziumoxid, Siliziumkarbid und Zirkonoxid.

3. Der Heizkörper (11) gemäss Anspruch 1, bei dem die Stärke der ersten Schutzfolies (113) im Bereich von 10 nm bis 1000 nm liegt.

4. Der Heizkörper (1) gemäss Anspruch 1, bei dem das Material der zweiten Schutzfolie (114) leitfähige Keramik oder Metall ist;
wobei, wenn das Material der zweiten Schutzfolie (114) die leitfähige Keramik ist, das Material der leitfähigen Keramik eins oder mehrere folgender Materialien: Titannitrid und Titandiborid umfasst.

5. Der Heizkörper (11) gemäss Anspruch 1, bei dem die Stärke der zweiten Schutzfolie (114) im Bereich von 10 nm bis 2000 nm liegt.

6. Der Heizkörper (11) gemäss Anspruch 1, bei dem das flüssigkeitsleitende Substrat (111) ein dichtes flüssigkeitleitendes Substrat (111) ist, und wobei das flüssigkeitleitende Substrat (111) weiter eine zweite Oberfläche (1112) umfasst, die der ersten Oberfläche (1111) gegenüber liegt, wobei das flüssigkeitsleitende Substrat (111) eine Vielzahl von ersten Mikroporen (1113) umfasst und die Vielzahl von ersten Mikroporen (1113) bestimmte Durchgangslöcher sind, die durch die erste Oberfläche (1111) und die zweite Oberfläche (1112) dringen;
wobei das Material des flüssigkeitsleitenden Substrats (111) Quarz, Glas oder dichte Keramikl ist; und die Vielzahl von ersten Mikroporen (1113) gerade Durchgangslöcher sind;
wobei die Heizmaterialschicht (112) und die erste Schutzfolie ( 113) sich in die Wandfläche eines jeden der vielzähligen ersten Mikroporen (1113) erstrecken.

7. Der Heizkörper (11) gemäss Anspruch 1, bei dem das Material des flüssigkeitsleitenden Substrats (111) poröse Keramik ist; und das flüssigkeitsleitendes Substrat (111) eine Vielzahl von unbestimmten Durchgangslöchern umfasst; oder
bei dem das flüssgikeitsleitende Substrat (111) stapelartig angeordnet eine poröse Keramikschicht und eine dichte Keramikschicht umfasst, wobei die dichte Keramikschicht eine Vielzahl von bestimmten geraden Durchgangslöchern senkrecht zur Richtung der Stärke des flüssigkeitsleitenden Substrats (111) umfasst, und die Heizmaterialschicht (112) auf der Oberfläche der dichten Keramikschicht abgewandt von der porösen Keramikschicht angeordnet ist.

8. Der Heizkörper (11) gemäs Anspruch 1, bei dem die Heizmaterialschicht (112) ein Heizfolie ist und die Stärke der Heizfolie im Bereich von 200 nm bis 5 µm liegt.

9. Der Heizkörper (11) gemäss Anspruch 1, bei dem die Stärke der Heizmaterialschicht (112) im Bereich von 5 µm bis 100 µm liegt und die Heizmaterialschicht (112) eine bedruckte Metallaufschwämmungsschicht ist.

10. Der Heizkörper (11) gemäss Anspruch 1, bei dem die erste Schutzfolie (113) den gesamten Heizteil(1121) und die zweite Schutzfolie (114) den gesamten Anschlussteil (1122) bedeckt.

11. Der Heizkörper (1) gemäss Anspruch 1, bei dem das flüssigkeitsleitende Substrat (111) die Form eines Zylinders aufweist, das flüssigkeitsleitende Substrat (111) eine Innenoberfläche und eine Aussenoberfläche umfasst und die Heizmaterialschicht (112) auf der Innenoberfläche oder der Aussenoberfläche angeordnet ist.

12. Der Heizkörper (11) gemäss Anspruch 1, bei dem die Heizmaterialschicht (112), die erste Schutzfolie (113) und die zweite Schutzfolie (114) auf der ersten Oberfläche (1111) des flüssigkeitsleitenden Substrats (111) geformt sind.

13. Der Heizkörper (11) gemäss Anspruch 1, bei dem der Anschlussteil (1122) der Heizmaterialschicht (112) und die zweite Schutzfolie (114) eine Elektrode bilden.

14. Eine Zerstäubungsgruppe (1) **dadurch gekennzeichnet, dass** sie Folgendes umfasst:
Einen Flüssigkeitsspeicherungshohlraum (14), der konfiguriert ist, um eine flüssige Aerosol erzeugende Substanz zu speichern; und
einen Heizkörper (11) gemäss irgendeinem der Ansprüche 1-13, und wobei der Heizkörper (11) in Fluidverbindung mit dem Flüssigkeitsspeicherungshohlraum (14) steht.

15. Eine elektronische Zerstäubungsvorrichtung (100), **dadurch gekennzeichnet dass**:
Sie eine Zerstäubungsgruppe (1) gemäss Anspruch 14 umfasst; und eine Stromversorgungsgruppe (2), die elektrisch an den Heizkörper (11) angeschlossen ist.

## Revendications

1. Corps chauffant (11), destiné à être utilisé sur un dispositif électronique de pulvérisation (100), qui est configuré pour pulvériser une substance générant un aérosol, dont le corps chauffant (H) comprend :
un substrat de guidage du liquide (111) ;
une couche de matériau chauffant (112), disposée sur une première surface (1111) du substrat de guidage de liquide (111), dans laquelle la couche de matériau chauffant (112) est un matériau de chauffage par résistance, et comprend :
une partie chauffante (1121), dans laquelle une portion du substrat de guidage de liquide (111), où la partie chauffante (1121) est disposée, est définie comme une région chauffante (a) ; et
une partie de connexion (1122), dans laquelle une autre portion du substrat de guidage de liquide (111), sur laquelle la partie de connexion (1122) est disposée, est définie comme une région d'électrode (b) ; **caractérisé par**
un premier film de protection (113), au moins partiellement disposé sur la surface de la partie chauffante (1121) à l'écart du substrat de guidage de liquide (111), dans lequel le matériau du premier film de protection (113) est non conducteur et résistant à la corrosion de la substance qui génère l'aérosol ; et
un deuxième film de protection (114), au moins partiellement disposé sur la surface de la partie de connexion (1122) à l'écart du substrat de guidage de liquide (111), dans lequel le matériau du deuxième film de protection (114) est conducteur et résistant à la corrosion de la substance qui génère l'aérosol.

2. Le corps chauffant (11) selon la revendication 1, dans lequel le premier film de protection (113) est fabriqué à partir d'un un matériau céramique ou du verre ;
dans lequel, lorsque le matériau constitutif du premier film de protection (113) est la céramique, le matériau constitutif de la céramique comprend un ou plusieurs des matériaux suivants : nitrure d'aluminium, nitrure de silicium, oxyde d'aluminium, oxyde de silicium, carbure de silicium et oxyde de zirconium.

3. Le corps chauffant (11) selon la revendication 1, dans lequel l'épaisseur du premier film de protection (113) est comprise entre 10 nm et 1000 nm.

4. Le corps chauffant (11) selon la revendication 1, dans lequel le matériau constitutif du second film de protection (114) est une céramique conductrice ou un métal ;
dans lequel, lorsque le matériau du second film de protection (114) est une céramique conductrice, le matériau de la céramique conductrice comprend un ou plusieurs nitrures de titane et diborure de titane.

5. Le corps chauffant (11) selon la revendication 1, dans lequel l'épaisseur du second film de protection (114) est comprise entre 10 nm et 2000 nm.

6. Le corps chauffant (11) selon la revendication 1, dans lequel le substrat de guidage de liquide (111) est un substrat de guidage de liquides denses (111), et le substrat de guidage de liquide (111) comprend en outre une deuxième surface (1112) opposée à la première surface (1111), le substrat de guidage de liquide (111) comprenant une pluralité de premiers micropores (1113), et la pluralité de premiers micropores (1113) sont conçus comme des trous traversants pénétrant à travers la première surface (1111) et la deuxième surface (1112) ;
où le matériau du substrat de guidage de liquide (111) comprend du quartz, du verre ou de la céramique dense, et la pluralité de premiers micropores (1113) sont des trous de passage rectilignes ;
où la couche de matériau chauffant (112) et le premier film de protection (113) s'étendent à travers la surface de la paroi de chacun des premiers micropores (1113).

7. Le corps chauffant (11) selon la revendication 1, dans lequel le matériau du substrat de guidage de liquide (111) est une céramique poreuse, et le substrat de guidage de liquide (111) comprend une pluralité de trous traversants non planifiés ; ou
le substrat de guidage des liquides (111) comprend une couche de céramique poreuse et une couche de céramique dense disposées de manière empilée, la couche de céramique dense comprenant une pluralité de trous de passage rectilignes planifiés qui sont perpendiculaires à la direction de l'épaisseur du substrat de guidage des liquides (111), et la couche de matériau chauffant (112) étant disposée sur la surface de la couche de céramique dense, à l'écart de la couche de céramique poreuse.

8. Le corps chauffant (11) selon la revendication 1, dans lequel la couche de matériau chauffant (112) est un film chauffant, et l'épaisseur du film chauffant est comprise entre 200 nm et 5 µn.

9. Le corps chauffant (11) selon la revendication 1, dans lequel l'épaisseur de la couche de matériau chauffant (112) est comprise entre 5 µm et 100 µm, et la couche de matériau chauffant (112) est une couche imprimée de matériau métallique.

10. Le corps chauffant (11) selon la revendication 1, dans lequel le premier film de protection (113) recouvre l'ensemble de la partie chauffante (1121), et le second film de protection (114) recouvre l'ensemble de la partie de connexion (1122).

11. Le corps chauffant (11) selon la revendication 1, dans lequel le substrat de guidage de liquide (111) a la forme d'un cylindre, le substrat de guidage de liquide (111) comprend une surface intérieure et une surface extérieure, et la couche de matériau chauffant (112) est disposée sur la surface intérieure ou la surface extérieure.

12. Le corps chauffant (11) selon la revendication 1, dans lequel la couche de matériau chauffant (112), le premier film de protection (113) et le second film de protection (114) sont formés sur la première surface (1111) du substrat de guidage de liquide (111).

13. Le corps chauffant (11) selon la revendication 1, dans lequel la partie de connexion (1122) de la couche de matériau chauffant (112) et le second film de protection (114) forment une électrode.

14. Dispositif d'atomisation (1), **caractérisé en ce qu'**il comprend :
une cavité de stockage de liquide (14), configurée pour stocker une substance liquide générant des aérosols ; et
un corps chauffant (11) selon une quelconque des revendications 1 à 13, dans lequel le corps de chauffage (11) est en communication fluidique avec la cavité de stockage de liquide (14).

15. Dispositif électronique d'atomisation (100), **caractérisé en ce qu'**il comprend : un bloc d'atomisation (1) selon la revendication 14 ; et
un bloc d'alimentation électrique (2), connecté électriquement au corps chauffant (11).
